# EUROPEAN PATENT SPECIFICATION

(11) **EP 2 512 520 B1**
(45) Date of publication and mention of the grant of the patent: **11.09.2013**
(21) Application number: 10810926.5
(22) Date of filing: 14.12.2010
(51) Int. Cl.: A61K 47/48, C08L 63/00, C08L 71/02, C08L 23/08, A61K 8/04, A61Q 9/02

(54) **POLYETHYLENE GLYCOL CONTAINING COMPOSITION FOR SHAVING**
POLYETHYLENGLYKOL ENTHALTENDE & xA;ZUSAMMENSETZUNG FÜR EIN RASIERHILFSMITTEL
COMPOSITION DE RASAGE CONTENANT DU POLYÉTHYLÈNE GLYCOL

(30) Priority: 18.12.2009 EP 09179791
(43) Date of publication of application: 24.10.2012
(73) Proprietor: Koninklijke Philips Electronics N.V., 5621 BA Eindhoven (NL)
(72) Inventor: HARVEY, Severin Luc Ramses, NL-5656 AE Eindhoven (NL); LELIEVELD, Mark Johannes, NL-5656 AE Eindhoven (NL); ZUIDERVAART, Jasper, NL-5656 AE Eindhoven (NL)
(74) Representative: Coops, Peter
(86) International application number: PCT/IB2010/055806
(87) International publication number: WO 2011/073906

(56) References cited:
- EP-A2- 0 152 292
- WO-A1-00/64500
- WO-A1-2006/015149
- US-A- 5 869 075
- US-A1- 2002 086 919
- US-A1- 2004 161 450
- US-A1- 2005 175 575

## Description

### FIELD OF THE INVENTION

The invention relates to a polyethylene glycol composition, to a container comprising such polyethylene glycol composition, to a human hair shaving apparatus comprising such container, as well as to the use of the polyethylene glycol composition.

### BACKGROUND OF THE INVENTION

Lubricant compositions for personal care are known in the art.

US-2008 210580, for instance, describes arming personal lubricant compositions and condom products including such compositions. Further, US-2008/210580 describes methods of making such condom products. According to US-2008/210580, the warming lubricant compositions include at least about 50 % by weight of a polyalkylene glycol component, preferably including at least two portions of different molecular weights, and an effective amount of a viscosity inducing component.

WO-2009/051486 describes components and methods for recovery of damaged skin and improving skin moisturisation. Presented is a shaving composition, particularly an emulsion, which comprises preferably a volatile hydrocarbon propellant. A volatile nitrogen base is incorporated in the shaving composition to bring the pH of the shaven skin to a desired value of from 4 to 5 directly after application to boost the skin repair properties. Preferably the shaving composition is soap-free, yet in the alkaline pH range so as to have the shaving advantages of soap and the skin care advantages of the pH control system. The shaving composition according to WO-2009/051486 can be used as a two-in-one products, such as in the two-in-one products shave & aftershave, deodorant & shave and shower & shave

Further, WO-2008/150155 describes a composition for shaving, for replacing, for example, a shaving foam or shaving gel during shaving. For this, WO-2008/150155 provides a composition comprising: a) 5-20 % (w/w) of one or more vegetable oils; b) 20-50 % (w/w) of a lower alkyl, branched or unbranched, comprising 1 to 4 carbon atoms; c) 1-10 % (w/w) of one or more structure providing compounds; and d) 40-65 % (w/w) water. Further, WO-2008/150155 describes a method for preparing such composition for use during shaving.

The aspect of the invention is to provide a friction reducing shaving lotion consisting of a composition comprising one or more low molecular weight polyethylene glycols (LMW-PEG) and one or more high molecular weight polyethylene glycols (HMW-PEG), wherein the one or more LMW-PEGs have a molecular weight in a range of 200 to 500 dalton, wherein the one or more HMW-PEGs have a molecular weight in a range of 8,000 to 45,000 dalton, and wherein the weight ratio of the one or more LMW-PEGs to the one or more HMW-PEGs is in the range of 2:1 to 8:1.

The invention is also directed to a container comprising:
a container volume containing said composition, and
a dispenser configured to dispense the composition from the container volume, wherein the container is configured to be attached to a shaving apparatus.

Further, the invention is directed to a
human hair shaving apparatus, comprising:
container said
wherein the apparatus and the dispenser of the container are configured to provide the composition, to the skin of a user while shaving with the human hair shaving apparatus.

The composition as defined herein may be used as (blade) shaving lotion, especially as friction reducing (blade) shaving lotion, but may also be used as condom lubricant.

### DETAILED DESCRIPTION OF THE EMBODIMENTS

As mentioned above, in a preferred embodiment the invention provides a composition comprising one or more low molecular weight polyethylene glycols (LMW-PEG) and one or more high molecular weight polyethylene glycols (HMW-PEG), wherein the one or more LMW-PEGs preferably have a molecular weight in a range of 200 to 500 dalton (especially PEG 200 to PEG 400) and wherein the HMW-PEG comprises one or more PEGs selected from the group consisting of PEG 8,000 and larger, especially wherein the one or more HMW-PEGs preferably have a molecular weight in a range of 8,000 to 45,000 dalton.

Polyethylene glycol (PEG) is also known as polyethylene oxide (PEO) or polyoxyethylene (POE). PEG, PEO or POE refers to an oligomer or polymer of ethylene oxide. Here, the abbreviation PEG is used, whereby the prefixes "LMW" and "HMW" herein refer to the above-mentioned molecular weight (MW) ranges. The numbers behind "PEG" indicate the molecular weight in dalton (as known in the art). PEG may be a liquid or low-melting solids, depending on their molecular weights. PEGs may be prepared by polymerization of ethylene oxide and are commercially available over a wide range of molecular weights from for instance 200 dalton to 10,000,000 dalton.

Herein, the PEGs may in an embodiment also be branched.

In an embodiment, HMW-PEG, optionally comprising a composition of different molecular weights, may be incorporated in a liquid PEG (low molecular weight) solution. The latter is herein also indicated as LMW-PEG. The HMW-PEG may be provided as a (fine) powder and is mixed with the LMW-PEG. In this way a suspension or gel may be provided. Such composition may be provided to the blade(s) (or alternative contact part(s)) of a shaver via a dispenser, or may be used in other applications.

The composition may optionally further comprise one or more of water; one or more vitamins; one or more fragrances; one or more odors; one or more surfactant(s); and one or more propellant(s).

The composition may optionally (also) further comprise one or more of thickening agents; sequestrants; vitamins (e.g. retinol); vitamin derivatives (e.g. tocophenyl actetate, niacinamide, panthenol); sunscreens; anti-wrinkle/ anti- atrophy actives (e.g. N-acetyl derivatives, thiols, hydroxyl acids, phenol); anti-oxidants (e.g. ascorbic acid derivatives, tocopherol); skin soothing agents/skin healing agents (e.g. panthenoic acid derivatives, aloe vera, allantoin); skin lightening agents (e.g. kojic acid, arbutin, ascorbic acid derivatives); skin tanning agents (e.g. dihydroxyacteone); polymeric phase structurant (e.g. naturally derived polymers, synthetic polymers, cross-linked polymers, block copolymers, copolymers, hydrophilic polymers, non-ionic polymers, anionic polymers, hydrophobic polymers, hydrophobic ally modified polymers, associative polymers, and oligomers); anti-acne medicaments; essential oils; sensates; pigments; colorants; pearlescent agents; and particles (e.g. talc, kaolin, mica, smectite clay, cellulose powder, polysiloxane, silicas, carbonates, titanium dioxide, polyethylene beads). Other optional ingredients are most typically those materials approved for use in cosmetics and that are described in the CTFA Cosmetic Ingredient Handbook, Second Edition, The Cosmetic, Toiletries, and Fragrance Association, Inc. 1988, 1992. The composition is especially skin tolerant.

Especially good results were obtained when the weight ratio (in the composition) of the one or more LMW-PEGs to the one or more HMW-PEG is in the range of 2:1 1 to 8:1, especially in the range of 3:1 1 to 5:1. Further, it appeared that good results could be obtained when the one or more LMW-PEGs have a molecular weight in a range of 200 to 400 dalton, like especially PEG 200 to PEG 350, like PEG 250 to PEG 300, and/or when the one or more HMW-PEGs have a molecular weight in a range of 30,000 dalton and larger, especially have a molecular weight in a range of 30,000 to 40,000 dalton. Even more preferred appears a composition wherein the weight ratio of the one or more LMW-PEGs to the one or more HMW-PEGs is in the range of 3:1 to 5:1, wherein the one or more LMW-PEGs have a molecular weight in a range of 200 to 400 dalton, and wherein the one or more HMW-PEGs have a molecular weight in a range of 30,000 dalton and larger, especially have a molecular weight in a range of 30,000 to 40,000 dalton.

In a further aspect, the invention provides a container comprising (1) a container volume containing the composition according to the invention, and (2) a dispenser configured to dispense the composition from the container volume. The dispenser can for instance be used to dispense the composition on the skin of a user, in or on condoms, for instance during production thereof, and or on the blade(s) (or other contact part(s) of a shaving apparatus (such as a on the blade(s) of a blade shaving apparatus).

The shaving apparatus can be an electric shaving apparatus or a non-electric shaving apparatus. Further, the shaving apparatus can be a shaving apparatus designed for man, or a shaving apparatus designed for woman (ladyshave). It can be a shaving apparatus designed to be used for beard and/or moustache shaving, or a shaving apparatus designed for shaving of arms, for legs, or for bikini-lines. The shaving apparatus can be designed to be used under a shower. The shaving apparatus is especially designed for cutting and/or removing hair on the skin of a human.

In a preferred embodiment (see also above), the weight ratio (in the composition) (in the container, or more precisely in the container volume) of the one or more LMW-PEGs to the one or more HMW-PEGs is in the range of 2:1 to 8:1, especially 3:1 to 5:1. Especially, the one or more LMW-PEGs have a molecular weight in a range of 200 to 400 dalton, such as PEG 200 to PEG 350, like PEG 200 to PEG 300. Further, especially the one or more HMW-PEGs have a molecular weight in a range of 30,000 to 40,000 dalton. In a preferred embodiment, the weight ratio in the composition (in the container, or more precisely in the container volume) of the one or more LMW-PEGs to the one or more HMW-PEGs is in the range of 3:1 to 5:1, wherein the one or more LMW-PEGs have a molecular weight in a range of 200 to 400 dalton, especially PEG 200 to PEG 350, such as PEG 200 to PEG 300, and wherein the one or more HMW-PEGs have a molecular weight in a range of 30,000 to 40,000 dalton.

In a further aspect, the invention provides a human hair shaving apparatus comprising a container such as defined above, wherein the apparatus and the dispenser of the container are configured to provide the composition to the skin of a user while shaving with the human hair shaving apparatus. In a specific embodiment, the human hair shaving apparatus is an electric human hair shaving apparatus, especially an electric blade shaving apparatus. The dispenser may be a manually controlled dispenser, but may also be a controller controlled dispenser. For instance, during shaving, the dispenser may provide a controlled amount of composition per time unit. Alternatively, a sensor may be applied, sensing the presence of the composition on a blade, and in control thereof, the dosage of composition may be controlled by the dispenser to the blade(s) (or alternatively, a sensor may be applied, sensing the presence of the composition on contact part(s), and in control thereof, the dosage of composition may be controlled by the contact part(s)). The phrase "the dosage of composition may be controlled" indicates that the amount (dosage) of composition provide by the dispenser is controlled. Is a (too) low amount of composition sensed, the dosage may be increased (temporarily), and would a (too) high amount of composition be sensed, the dosage may be reduced (temporarily).

The composition of the invention may be used in different applications. In a specific aspect, the composition may be used as shaving lotion, especially as friction reducing shaving lotion. In yet another embodiment, the composition may be used as condom lubricant. In yet another embodiment, the composition may also be used for other applications, such as an erotic gel; see further also above.

### Examples

A number of PEG additive combinations ("compositions") were tested on the skin to investigate their frictional properties. The friction was measured using the rotating friction meter form Courage and Khazaka (see for instance, http://www.courage-khazaka.de/, and especially http://www.courage-khazaka.de/download/pdf/studies_frictiometer.pdf, wherein it is amongst others indicated that: "the state and function of human skin can be quantified by numerous non-invasive test methods). There are, however, still no valid methods to measure the tactile properties of the skin surface and thus to quantify the state of the skin on the one hand, and to determine the negative and positive effects of tactile influences on the other hand. The measuring device (Frictiometer) consists of a sensor, a steering unit and a monitor. The torque, the circular friction on the skin surface, is measured via the motor load current and is shown as a voltage drop. The Frictiometer® FR 770 (Product of the month, Frictiometer® FR 770, SÖFW Journal April 2005.) is integrated into the CK Multiprobe Adapter System MPA and supplies another non-invasive test method for the human skin. Measurement of the smoothness condition of the skin." See also H. Tronnier, M. Wiebusch, U. Heinrich, Frictiometry on human skin, in a poster presentation, DermaDays Universität Witten-Herdecke, April 2005).

Twenty seven different solutions were made (see table below). Sample numbers 11, 12, 21, 20 were especially rather nice and creamy.

| No | Water (gram) | PEG 35,000 (gram) | PEG 6,000 (gram) | PEG 200 (gram) |
|---|---|---|---|---|
| 1 * | 0 | 0 | 0 | 20 |
| 2 * | 0 | 0 | 2 | 18 |
| 3 * | 0 | 0 | 4 | 16 |
| 4 * | 0 | 2 | 0 | 18 |
| 5 | 0 | 2 | 2 | 16 |
| 6 | 0 | 2 | 4 | 14 |
| 7 | 0 | 4 | 0 | 16 |
| 8 | 0 | 4 | 2 | 14 |
| 9 | 0 | 4 | 4 | 12 |
| 10 * | 2 | 0 | 0 | 18 |
| 11 * | 2 | 0 | 2 | 16 |
| 12 * | 2 | 0 | 4 | 14 |
| 13 | 2 | 2 | 0 | 16 |
| 14 | 2 | 2 | 2 | 14 |
| 15 | 2 | 2 | 4 | 12 |
| 16 | 2 | 4 | 0 | 14 |
| 17 | 2 | 4 | 2 | 12 |
| 18 | 2 | 4 | 4 | 10 |
| 19 * | 4 | 0 | 0 | 16 |
| 20 * | 4 | 0 | 2 | 14 |
| 21 * | 4 | 0 | 4 | 12 |
| 22 | 4 | 2 | 0 | 14 |
| 23 | 4 | 2 | 2 | 12 |
| 24 | 4 | 2 | 4 | 10 |
| 25 | 4 | 4 | 0 | 12 |
| 26 | 4 | 4 | 2 | 10 |
| 27 | 4 | 4 | 4 | 8 |

| | | | | |
|---|---|---|---|---|
| * not according to the invention | | | | |

The samples were tested and it appeared that some of the samples 19 and up were not (very good) pumpable because substantially solid compositions were obtained. From this it can be concluded that the PEGs are preferably combined to provide a composition that is still flowable. Preferably, the viscosity at room temperature is in the range of about 2 to 5.10⁴ mPa.s, even more especially 2 to 5.10³ mPa.s, like 4 to 1,000 mPa.s, especially 5 to 500 mPa.s (at a shear rate of about 1 to 100 s⁻¹, such as 20 s⁻¹).

The protocol for the friction measurement is as follows: measurement are done on the arm; always at the same location. An amount of about 0.1 ml of water is added to the measurement site and about 0.1 ml of additive is also added. Then, the shaving probe is applied, so that it is perpendicular to the skin.

Different types of additive (i.e. composition) were further tested:
1 PEG 200 (liquid)
2 2 g PEG 6,000 + 18 g PEG 200
3 4 g PEG 6,000 + 16 g PEG 200
4 2 g PEG 35,000 + 18 g PEG 200
5 2 g PEG 35,000 + 2 g PEG 6,000 + 16 g PEG 200
7 4 g PEG 35,000 + 16 g PEG 200

It appears that the friction of those compositions is in the same order of magnitude as those of commercially available compositions, such as HS800 (containing in concentrations < 5 % PEG 45M, C13-16 Isoparaffin, Sodium Acrylates, C10-30 Alkyl Acrylate Crosspolymer). It further appears that adding higher molecular weight PEG to a PEG solution will decrease the value of the friction measured by the friction probe. It seems that the higher the PEG molecular weight the more friction reduction is achieved. The highest drop in friction achieved by adding higher molecular weight PEG is almost 40 %.

It further appears that the volume of the compositions according to the invention is much smaller than the volume of commercially available compositions, such as HS800. In comparison to HS800, 10 to 20 times less volume of liquid composition is needed to get the substantially the same friction. Hence, the compositions according to the invention can advantageously be used in a container of a human hair shaving apparatus.

Such container may be detachably attached to the shaving apparatus. In this way, the container can be refilled or replaced with a filled container after use.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. In the device claim enumerating several means, several of these means may be embodied by one and the same item of hardware. The mere fact that certain measures are recited in mutually different dependent claims does not indicate that a combination of these measures cannot be used to advantage.

## Claims

1. A friction reducing shaving lotion consisting of a composition comprising one or more low molecular weight polyethylene glycols (LMW-PEG) and one or more high molecular weight polyethylene glycols (HMW-PEG), wherein the one or more LMW-PEGs have a molecular weight in a range of 200 to 500 dalton, wherein the one or more HMW-PEGs have a molecular weight in a range of 8,000 to 45,000 dalton, and wherein the weight ratio of the one or more LMW-PEGs to the one or more HMW-PEGs is in the range of 2:1 to 8:1.

2. The composition according to claim 1, wherein the one or more LMW-PEGs have a molecular weight in a range of 200 to 400 dalton.

3. The composition according to any one of the preceding claims, wherein the one or more HMW-PEGs have a molecular weight in a range of 30,000 to 40,000 dalton.

4. The composition according to any one of the preceding claims, wherein the weight ratio of the one or more LMW-PEGs to the one or more HMW-PEGs is in the range of 3:1 to 5:1, wherein the one or more LMW-PEGs have a molecular weight in a range of 200 to 400 dalton, and wherein the one or more HMW-PEGs have a molecular weight in a range of 30,000 to 40,000 dalton.

5. A container comprising:
- a container volume containing a composition according to claim 1, and
- a dispenser configured to dispense the composition from the container volume, wherein the container is configured to be attached to a shaving apparatus.

6. The container according to claim 5, wherein the container is configured such that the container may detachably be arranged to the shaving apparatus.

7. The container according to claim 5 wherein the weight ratio of the one or more LMW-PEGs to the one or more HMW-PEGs is in the range of 2:1 to 8:1.

8. The container according to any one of the claims 5 to 7, wherein the one or more LMW-PEGs have a molecular weight in a range of 200 to 400 dalton.

9. The container according to any one of the claims 5 to 8, wherein the one or more HMW-PEGs have a molecular weight in a range of 30,000 to 40,000 dalton.

10. The container according to any one of the claims 5 to 9, wherein the weight ratio of the one or more LMW-PEGs to the one or more HMW-PEGs is in the range of 3:1 to 5:1, wherein the one or more LMW-PEGs have a molecular weight in a range of 200 to 400 dalton, and wherein the one or more HMW-PEGs have a molecular weight in a range of 30,000 to 40,000 dalton.

11. A human hair shaving apparatus, comprising:
- a container according to any one of claims 5 to 10,
wherein the apparatus and the dispenser of the container are configured to provide the composition, according to any one of claims 1 to 4, to the skin of a user while shaving with the human hair shaving apparatus.

12. The human hair shaving apparatus, wherein the human hair shaving apparatus is an electric blade shaving apparatus.

13. Use of the composition according to any one of claims 1 to 4 as shaving lotion.

14. Use of the composition according to any one of claims 1 to 4 as friction reducing shaving lotion.

15. Use of the composition according to any one of claims 1 to 5 as condom lubricant.

## Patentansprüche

1. Reibungsmindemde Rasierlotion, die aus einer Zusammensetzung mit einem oder mehreren niedermolekularen Polyethylenglycolen (LMW-PEG) und einem oder mehreren hochmolekularen Polyethylenglycolen (HMW-PEG) besteht, wobei das eine oder die mehreren LMW-PEGs ein Molekulargewicht in einem Bereich von 200 bis 500 Dalton aufweisen, wobei das eine oder die mehreren HMW-PEGs ein Molekulargewicht in einem Bereich von 8.000 bis 45.000 Dalton aufweisen, und wobei das Gewichtsverhältnis des einen oder der mehreren LMW-PEGs zu dem einen oder der mehreren HMW-PEGs in dem Bereich von 2:1 bis 8:1 liegt.

2. Zusammensetzung nach Anspruch 1, wobei das eine oder die mehreren LMW-PEGs ein Molekulargewicht in einem Bereich von 200 bis 400 Dalton aufweisen.

3. Zusammensetzung nach einem der vorangegangenen Ansprüche, wobei das eine oder die mehreren HMW-PEGs ein Molekulargewicht in einem Bereich von 30.000 bis 40.000 Dalton aufweisen.

4. Zusammensetzung nach einem der vorangegangenen Ansprüche, wobei das Gewichtsverhältnis des einen oder der mehreren LMW-PEGs zu dem einen oder der mehreren HMW-PEGs in dem Bereich von 3:1 bis 5:1 liegt, wobei das eine oder die mehreren LMW-PEGs ein Molekulargewicht in einem Bereich von 200 bis 400 Dalton aufweisen, und wobei das eine oder die mehreren HMW-PEGs ein Molekulargewicht in einem Bereich von 30.000 bis 40.000 Dalton aufweisen.

5. Behälter mit:
- einem Behältervolumen, das eine Zusammensetzung nach Anspruch 1 enthält, sowie
- einem Spender, der so ausgeführt ist, dass er die Zusammensetzung aus dem Behältervolumen abgibt, wobei der Behälter so ausgeführt ist, dass er an einem Rasierapparat befestigt ist.

6. Behälter nach Anspruch 5, wobei der Behälter so ausgeführt ist, dass er an dem Rasierapparat abnehmbar angeordnet sein kann.

7. Behälter nach Anspruch 5, wobei das Gewichtsverhältnis des einen oder der mehreren LMW-PEGs zu dem einen oder der mehreren HMW-PEGs in dem Bereich von 2:1 bis 8:1 liegt.

8. Behälter nach einem der Ansprüche 5 bis 7, wobei das eine oder die mehreren LMW-PEGs ein Molekulargewicht in einem Bereich von 200 bis 400 Dalton aufweisen.

9. Behälter nach einem der Ansprüche 5 bis 8, wobei das eine oder die mehreren HMW-PEGs ein Molekulargewicht in einem Bereich von 30.000 bis 40.000 Dalton aufweisen.

10. Behälter nach einem der Ansprüche 5 bis 9, wobei das Gewichtsverhältnis des einen oder der mehreren LMW-PEGs zu dem einen oder der mehreren HMW-PEGs in dem Bereich von 3:1 bis 5:1 liegt, wobei das eine oder die mehreren LMW-PEGs ein Molekulargewicht in einem Bereich von 200 bis 400 Dalton aufweisen, und wobei das eine oder die mehreren HMW-PEGs ein Molekulargewicht in einem Bereich von 30.000 bis 40.000 Dalton aufweisen.

11. Rasierapparat zur Rasur von Haaren eines Menschen, mit:
- einem Behälter nach einem der Ansprüche 5 bis 10,
wobei der Apparat und der Spender des Behälters so ausgeführt sind, dass sie der Haut eines Benutzers die Zusammensetzung nach einem der Ansprüche 1 bis 4 während des Rasierens mit dem Rasierapparat zur Rasur menschlicher Haare zuführen.

12. Rasierapparat zur Rasur von Haaren eines Menschen, wobei es sich bei diesem um einen elektrischen Klingenrasierapparat handelt.

13. Verwendung der Zusammensetzung nach einem der Ansprüche 1 bis 4 als Rasierlotion.

14. Verwendung der Zusammensetzung nach einem der Ansprüche 1 bis 4 als reibungsmindemde Rasierlotion.

15. Verwendung der Zusammensetzung nach einem der Ansprüche 1 bis 5 als Kondom-Gleitmittel.

## Revendications

1. Lotion de rasage à réduction de frottement constituée d'une composition comprenant au moins un polyéthylène glycol de faible poids moléculaire (LMW-PEG) et au moins un polyéthylène glycol de poids moléculaire élevé (HMW-PEG), dans laquelle l'au moins un LMW-PEG a un poids moléculaire dans une plage de 200 à 500 daltons, dans laquelle l'au moins un HMW-PEG a un poids moléculaire dans une plage de 8 000 à 45 000 daltons, et dans laquelle le rapport de poids de l'au moins un LMW-PEG sur l'au moins un HMW-PEG est dans la plage de 2:1 à 8:1.

2. Composition selon la revendication 1, dans laquelle l'au moins un LMW-PEG a un poids moléculaire dans une plage de 200 à 400 daltons.

3. Composition selon l'une quelconque des revendications précédentes, dans laquelle l'au moins un HMW-PEG a un poids moléculaire dans une plage de 30 000 à 40 000 daltons.

4. Composition selon l'une quelconque des revendications précédentes, dans laquelle le rapport de poids de l'au moins un LMW-PEG sur l'au moins un HMW-PEG est dans la plage de 3:1 à 5:1, dans laquelle l'au moins un LMW-PEG a un poids moléculaire dans une plage de 200 à 400 daltons, et dans laquelle l'au moins un HMW-PEG a un poids moléculaire dans une plage de 30 000 à 40 000 daltons.

5. Récipient comprenant:
- un volume de récipient contenant une composition selon la revendication 1, et
- un distributeur configuré pour distribuer la composition à partir du volume de récipient, dans lequel le récipient est configuré pour être attaché à un appareil de rasage.

6. Récipient selon la revendication 5, dans lequel le récipient est configuré de telle sorte que le récipient puisse être agencé de manière détachable de l'appareil de rasage.

7. Récipient selon la revendication 5, dans lequel le rapport de poids de l'au moins un LMW-PEG sur l'au moins un HMW-PEG est dans la plage de 2:1 à 8:1.

8. Récipient selon l'une quelconque des revendications 5 à 7, dans lequel l'au moins un LMW-PEG a un poids moléculaire dans une plage de 200 à 400 daltons.

9. Récipient selon l'une quelconque des revendications 5 à 8, dans lequel l'au moins un HMW-PEG a un poids moléculaire dans une plage de 30 000 à 40 000 daltons.

10. Récipient selon l'une quelconque des revendications 5 à 9, dans lequel le rapport de poids de l'au moins un LMW-PEG sur l'au moins un HMW-PEG est dans la plage de 3:1 à 5:1, dans lequel l'au moins un LMW-PEG a un poids moléculaire dans une plage de 200 à 400 daltons, et dans lequel l'au moins un HMW-PEG a un poids moléculaire dans une plage de 30 000 à 40 000 daltons.

11. Appareil de rasage de poils humains, comprenant :
- un récipient selon l'une quelconque des revendications 5 à 10,
dans lequel l'appareil et le distributeur du récipient sont configurés pour fournir la composition, selon l'une quelconque des revendications 1 à 4, sur la peau d'un utilisateur pendant le rasage avec l'appareil de rasage de poils humains.

12. Appareil de rasage de poils humains, dans lequel l'appareil de rasage de poils humains est un appareil de rasage à lame électrique.

13. Utilisation de la composition selon l'une quelconque des revendications 1 à 4 en tant que lotion de rasage.

14. Utilisation de la composition selon l'une quelconque des revendications 1 à 4 en tant que lotion de rasage à réduction de frottement.

15. Utilisation de la composition selon l'une quelconque des revendications 1 à 5 en tant que lubrifiant de préservatif.
